# EUROPÄISCHE PATENTSCHRIFT

(11) **EP 2 260 051 B1**
(45) Veröffentlichungstag und Bekanntmachung des Hinweises auf die Patenterteilung: **17.10.2012**
(21) Anmeldenummer: 09729525.7
(22) Anmeldetag: 17.03.2009
(51) Int. Cl.: C07H 7/04, C07H 7/06, C07D 309/10, C07D 405/04, C07D 407/04, A61K 31/351, A61P 3/10

(54) **GLUCOPYRANOSIDDERIVATE**
GLUCOPYRANOSIDE DERIVATIVES
DÉRIVÉS DE GLUCOPYRANOSIDE

(30) Priorität: 07.04.2008 DE 102008017590
(43) Veröffentlichungstag der Anmeldung: 15.12.2010
(73) Patentinhaber: Merck Patent GmbH, 64293 Darmstadt (DE)
(72) Erfinder: TSAKLAKIDIS, Christos, 69469 Weinheim (DE); BEIER, Norbert, 64354 Reinheim (DE)
(86) Internationale Anmeldenummer: PCT/EP2009/001946
(87) Internationale Veröffentlichungsnummer: WO 2009/124638

(56) Entgegenhaltungen:
- WO-A-2005/012318

## Beschreibung

Die Erfindung betrifft Verbindungen der Formel I 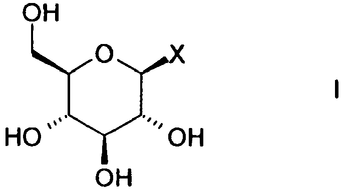 worin
- X: 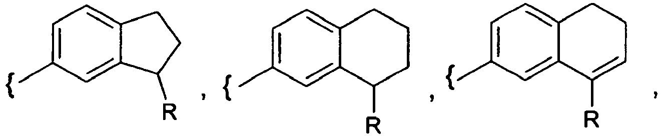 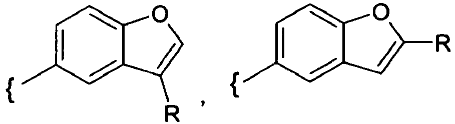 oder 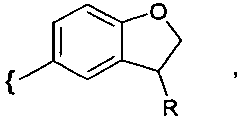 wobei der 6-gliedrige aromatische Ring ein- oder zweifach durch Hal, OR⁵, NR⁵R⁶, CN, COOR⁵, CONR⁵R⁶, -OCOA, NR⁵COR⁶ und/oder NR⁵SO₂A substituiert sein kann,
- R: Carb, Ar oder Het,
- R⁵, R⁶: jeweils unabhängig voneinander H oder A,
- Carb: Cycloalkyl mit 3-7 C-Atomen,
- Ar: unsubstituiertes oder ein-, zwei-, drei-, vier- oder fünffach durch Hal, A, Benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)pA und/oder -(CR⁵R⁶)ₘ-COOR⁵ substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der ein-, zwei- oder dreifach durch Hal, A, Benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)ₚA, -(CR⁵R⁶)ₘ-COOR⁵, =S, =NR¹ und/oder =O (Carbonylsauerstoff) substituiert sein kann,
- A: unverzweigtes oder verzweigtes Alkyl mit 1-10 C-Atomen, worin eine, zwei oder drei CH₂-Gruppen unabhängig voneinander durch O, S, SO, SO₂, NR⁵, -C=C- und/oder durch -CH=CH-Gruppen und/oder worin auch 1-7 H-Atome durch F und/oder CI ersetzt sein können, oder Cycloalkyl mit 3-7 C-Atomen,
- Hal: F, Cl, Br oder I,
- m: 0, 1, 2 oder 3,
- p: 0, 1 oder 2,
bedeuten,
sowie ihre pharmazeutisch verwendbaren Salze, Tautomere und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Sie zeigen SGLT1 und SGLT2 (sodium dependent glucose co-transporter) inhibierende Eigenschaften und können daher zur Bekämpfung und Verhütung von Diabetes vom Typ 1 und Typ 2 eingesetzt werden.

Die Absorption von Glucose im Bürstensaum des Dünndarms und in den proximalen Nierentubuli gegen einen Konzentrationsgradienten erfolgt über epitheliale natriumabhängige Glucose-Cotransporter (SGLTs). Es wurden mindestens zwei größere Klassen von SGLTs beschrieben: SGLT1 (beispielsweise Lee W.S. et al. (1994) The high-affinity Na+/Glucose co-transporter: reevaluation of function and distribution of expression. J. Biol. Chem. 269, 12032-12039) und SGLT2 (beispielsweise Mackenzie B. et al. (1994) SAAT1 ist a low-affinity Na+/glucose cotransporter and not an amino acid transporter. J. Biol. Chem. 269, 22488-22491).

Es wird angenommen, dass SGLT1 für die Absorption von Glucose im Darm wichtig ist, wohingegen SGLT2 wahrscheinlich für die Reabsorption von frei filtrierter Glucose in der Niere hauptsächlich verantwortlich ist.

Die hauptsächliche Veränderung bei Diabetes mellitus ist Hyperglykämie. Dies ist nicht nur ein Symptom der Erkrankung, sondern auch ein potentieller pathogener Faktor, der zu multiplen chronischen diabetischen mikro- und makrovaskularen Komplikationen und einer Störung der Insulinsekretion und Empfindlichkeit führt (Klein R. (1995), Hyperglycemia and microvascular and macrovascular disease in diabetes, Diabetes Care 18, 258-268; Rossetti L. (1995), Glucose toxicity: the implications of hyperglycemia in the pathophysiology of diabetes mellitus, Clin. Invest. Med. 18, 255-260). Somit ist beim Diabetes-Patient die ausschließliche Regulation der Blut-Glucosespiegel innerhalb des normalen Bereichs ein wichtiges Therapieziel. Entsprechend ihrer beschriebenen Funktion führt eine Hemmung der SGLTs zu einer verringerten Absorption und gesteigerten Ausscheidung von Glucose, sowie zu einer anschließenden Abnahme der Blut-Glucosespiegel. Somit kann die Unterdrückung der SGLTs eine geeignete Alternative zur Behandlung von Diabetes sein.

In der Literatur sind mehrere Substanzklassen mit SGLT-Wirkung beschrieben. All diesen Strukturen diente als Leitbild der Naturstoff Phlorizin.

Aromatische Glycosidderivate kennt man aus WO 2004/052902 und WO 2004/052903. Propiophenonglycoside sind beschrieben in WO 0280936, WO 0280935, JP 2000080041 und EP 850948. Glucopyranoslyoxybenzylbenzole sind in WO 0244192, WO 0228872 und WO 0168660 beschrieben. Glucopyranosyloxy-pyrazole kennt man aus WO 0268440, WO 0268439, WO 0236602 und WO 0116147. O-Glycosidbenzamide sind in WO 0174835 und WO 0174834 offenbart. C-Arylglycoside sind in WO 0127128 und US 2002137903 beschrieben. Alle bekannten Strukturen enthalten als sehr wichtiges Strukturelement die Glucose. Ferner sind aus US 2002/132807 Diarylsulfid-Verbindungen zur Behandlung von Entzündungs- und Immun-Erkrankungen bekannt. In EP 0 953 357 A1 werden allgemein Glycosid-Verbindungen als renale Drug-Carrier und in WO 95/23780 4-Hydroxy-phenoxy-hetero-cycloalkyl-Verbindungen als Hautaufheller beschrieben.

Aus der WO 2005/012318 A sind andere aromatische C-Glucoside bekannt, die Behandlung von Diabetes des Typ 1 und Typ 2 eingesetzt werden können.

Die erfindungsgemäßen Verbindungen weisen ein hohes Splitting in bezug auf die gewünschte Affinität von SGLT₂ zu SGLT₁ auf.

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.

Die Verbindungen der Formel I eignen sich weiterhin zur Prävention und Behandlung von diabetischen Spätschäden, wie z.B. Nephropathie, Retinopathie, Neuropathie sowie Syndrom X, Obesitas, Herzinfarkt, myocardialem Infarkt, peripheren arteriellen Verschlusskrankheiten, Thrombosen, Arteriosklerose, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie zur Prävention und Behandlung 15 von diabetischen Spätschäden, Syndrom X und Obesitas.

Die Verbindungen der Formel I können als Arzneimittelwirkstoffe in der Human- und Veterinärmedizin eingesetzt werden, insbesondere zur Behandlung und Verhütung von Diabetes vom Typ 1 und Typ 2.

Gegenstand der Erfindung sind die Verbindungen der Formel I und ihre Salze sowie ein Verfahren zur Herstellung von Verbindungen der Formel I sowie ihrer pharmazeutisch verwendbaren Derivate, Solvate, Salze und Stereoisomere, dadurch gekennzeichnet, daß man aus einer Verbindung der Formel II 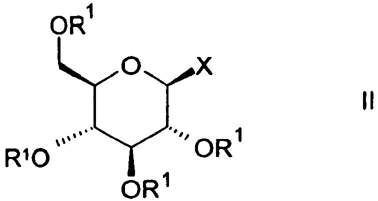 worin
X die in Anspruch 1 angegebene Bedeutung hat,
und R¹ eine Hydroxyschutzgruppe bedeutet,
R¹ abspaltet,
und/oder
eine Base oder Säure der Formel I in eines ihrer Salze umwandelt.

Unter Verbindungen der Formel I versteht man auch die Hydrate und Solvate dieser Verbindungen, ferner pharmazeutisch verwendbare Derivate.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Für alle Reste, die mehrfach auftreten, gilt, daß deren Bedeutungen unabhängig voneinander sind.

Vor- und nachstehend hat der Rest X die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.

A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m - oder p-Aminophenyl, o-, m- oder p-(N-Methylamino)-phenyl, o-, m- oder p-(N-Methylaminocarbonyl)-phenyl, o-, m- oder p-Acetamidophenyl, o-, m - oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m - oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Methylsulfanylphenyl, o-, m- oder p-Cyanphenyl, o-, m- oder p-Carboxyphenyl, o-, m- oder p-Methoxycarbonylphenyl, o-, m- oder p-Formylphenyl, o-, m- oder p-Acetylphenyl, o-, m- oder p-Aminosulfonylphenyl, o-, m- oder p-(Morpholin-4-ylcarbonyl)-phenyl, o-, m- oder p-(Morpholin-4-ylcarbonyl)-phenyl, o-, m- oder p-(3-Oxo-morpholin-4-yl)-phenyl, o-, m- oder p-(Piperidinyl-carbonyl)-phenyl, o-, m- oder p-[2-(Morpholin-4-yl)ethoxyJ-phenyl, o-, m- oder p-[3-(N,N-Diethylamino)propoxy]-phenyl, o-, m- oder p-[3-(3-Diethylaminopropyl)-ureido]-phenyl, o-, m- oder p-(3-Diethylamino-propoxy-carbonylamino)-phenyl, weiter bevorzugt 2,3-, 2,4-, 2,5-, 2,6-, 3,4-oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amine-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet besonders bevorzugt unsubstituiertes Phenyl, weiterhin vorzugsweise z.B. durch A, Hal, OA und/oder OH mono-, di- oder trisubstituiertes Phenyl.

Het bedeutet, ungeachtet weiterer Substitutionen, z.B. 2- oder 3-Furyl, 2 - oder 3-Thienyl, 1-, 2- oder 3-Pyrrolyl, 1-, 2, 4- oder 5-Imidazolyl, 1-, 3-, 4 - oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7 - oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.

Die heterocyclischen Reste können auch teilweise oder vollständig hydriert sein.

Ungeachtet weiterer Substitutionen kann Het also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder-5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3 - oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7 - oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

Het bedeutet besonders bevorzugt Pyridyl, Pyrimidinyl, Furyl, Thienyl, Tetrahydrofuranyl, Tetrahydropyranyl, Dioxolanyl, Pyrrolidinyl, Piperidinyl, Morpholinyl oder Piperazinyl, die auch einfach durch Hal, A und/oder =O (Carbonylsauerstoff) substituiert sein können.

R⁵, R⁶ bedeuten vorzugsweise, jeweils unabhängig voneinander, H oder CH₃.

Hal bedeutet vorzugsweise F, CI oder Br, aber auch I.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Einige bevorzugte Gruppen von Verbindungen können durch die folgenden Teilformeln Ia bis If ausgedrückt werden, die der Formel I entsprechen und worin die nicht näher bezeichneten Reste die bei der Formel I angegebene Bedeutung haben, worin jedoch
- in Ia R⁵, R⁶: jeweils unabhängig voneinander H oder Methyl, bedeuten;
- in Ib R: Ar bedeutet;
- in Ic R⁵, R⁶: jeweils unabhängig voneinander H oder CH₃ bedeuten;
- in Id Ar: unsubstituiertes oder ein-, zwei- oder dreifach durch Hal, A und/oder OR⁵ substituiertes Phenyl bedeutet;
- in Ie A: unverzweigtes oder verzweigtes Alkyl mit 1-6 C-Atomen, worin 1-7 H-Atome durch F und/oder Cl ersetzt sein können, bedeutet;
- in If: X 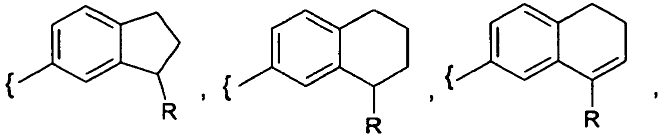 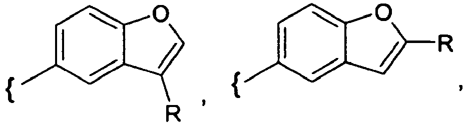 oder 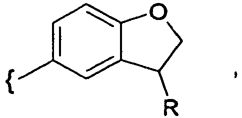
R Ar,
R⁵ H oder A,
Ar unsubstituiertes oder ein-, zwei- oder dreifach durch Hal, A und/oder OR⁵ substituiertes Phenyl,
A unverzweigtes oder verzweigtes Alkyl mit 1-6 C-Atomen, worin 1-7 H-Atome durch F und/oder Cl ersetzt sein können,
Hal F, Cl, Br oder I
bedeuten;
sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Der Ausdruck "Hydroxyschutzgruppe" ist allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl oder Silylschutzgruppen, wobei Benzyl und tert.-Butyl besonders bevorzugt sind.

Silyl bedeutet in der Regel Trimethyl-, Triethyl-, Trüsopropyl-, tert.-Butyl-dimethyl- oder t.-Butyl-diphenylsilyl, insbesondere Trimethyl- oder tert.-Butyl-dimethylsilyl.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Verbindungen der Formel II lassen sich vorzugsweise dadurch herstellen, dass man aus einer Verbindung der Formel III, 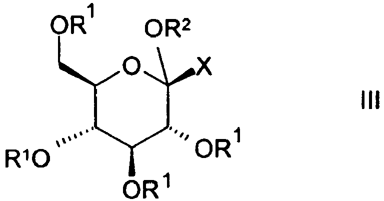
in der X die in Anspruch 1 angegebene Bedeutung hat,
R¹ eine Hydroxyschutzgruppe bedeutet
und R² H oder Methyl bedeutet,
die OR²-Gruppe am anomeren C-Atom der Glucose entfernt.

Verbindungen der Formel III lassen sich vorzugsweise dadurch herstellen, dass man eine Verbindung der Formel IV, 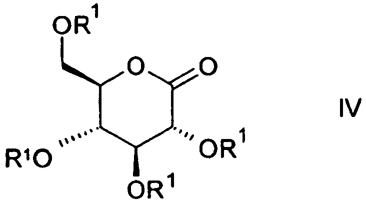 in der R¹ eine Hydroxyschutzgruppe bedeutet,
mit einer Verbindung der Formel V

M-X V

worin M ein Metall, vorzugsweise Lithium oder Magnesium, bedeutet, und X die in Anspruch 1 angegebene Bedeutung hat,
umsetzt.

Die Umsetzung erfolgt in einem inerten Lösungsmittel unter Standardbedingungen.

Verbindungen der Formel IV sind bekannt und lassen sich auch käuflich erwerben.

Verbindungen der Formel V erhält man vorzugsweise, indem man eine Verbindung der Formel VI

W-X VI

worin W CI, Br oder I bedeutet, und X die in Anspruch 1 angegebene Bedeutung hat,
mit einem einem Metall, vorzugsweise Magnesium, oder Metallorganyl, vorzugsweise Magnesium- oder Lithiumorganyl, umsetzt.

Magnesium- oder Lithiumorganyl bedeuten in der Regel Methyl-, Ethyl - oder Isopropylmagnesiumchlorid, Diethyl-oder Düsopropylmagnesium, n-Butyl-, sec.-Butyl- oder tert.-Butyllithium, insbesondere Isopropylmagnesiumchlorid oder tert.-Butyllithium.

Verbindungen der Formel VI, worin
X 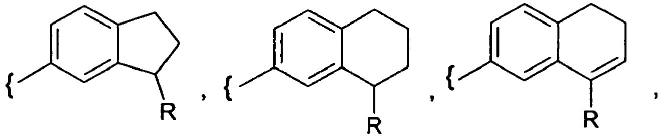 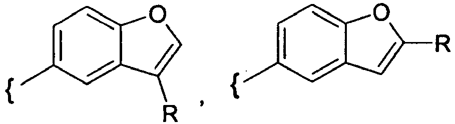 oder 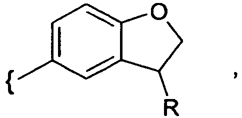 bedeutet,
erhält man vorzugsweise durch Umsetzung einer Verbindung der Formel VII

W-X VII

worin W CI, Br oder I bedeutet,
und X 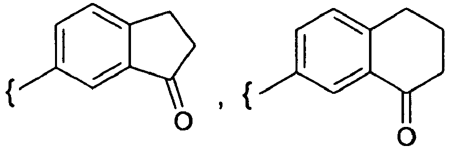 oder 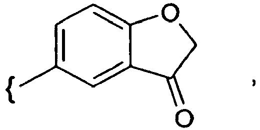 bedeutet,
mit einer Verbindung der Formel VIII

M-R VIII

worin M ein Metall, vorzugsweise Mg oder Li,
und R die in Anspruch 1 angegebene Bedeutung hat,
zu einer Verbindung der Formel IX

W-X IX

worin W CI, Br oder I bedeutet und
X 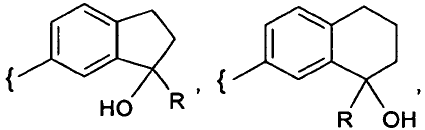 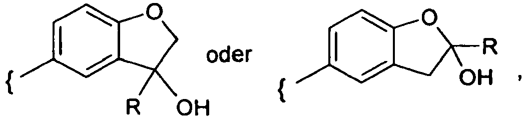 bedeutet,
umsetzt,
und anschließend die benzylische Hydroxygruppe nach literaturbekannten Methoden entfernt.

Verbindungen der Formel VI, worin
X 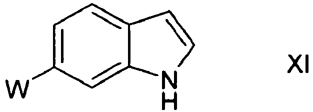 bedeutet,
erhält man vorzugsweise durch Umsetzung einer Verbindung der Formel XI 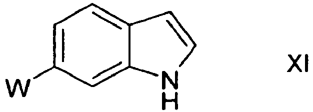 worin X Cl, Br oder I bedeutet,
mit einer Verbindung der Formel XII

W-R XII

worin R die in Anspruch 1 angegebene Bedeutung hat und
W Cl, Br oder I bedeutet,
umsetzt.

Verbindungen der Formel VII sind vorzugsweise durch intramolekulare Acylierung oder Alkylierung nach Standardmethoden erhältlich.

Die Reaktionszeiten der beschriebenen Methoden liegten je nach den angewendeten Bedingungen zwischen einigen Minuten und 14 Tagen, die Reaktionstemperatur zwischen etwa 0° und 150°, normalerweise zwischen 5° und 90°, besonders bevorzugt zwischen 10° und 70°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Hydrogenolytisch entfernbare Schutzgruppen (z. B. CBZ, Benzyl), Doppelbindungen oder Hydroxy- bzw. Alkoxy-gruppen in benzylischer Position, wie diese z.B. in Formeln III, IX oder X vorkommen, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Doppelbindungen oder Hydroxy- bzw. Alkoxy-gruppen in benzylischer Position, wie diese z.B. in Formeln III, IX oder X vorkommen; können weiterhin z. B. durch Behandeln mit einem Trialkylsilan wie Triethyl- oder Triisopropylsilan und einer Säure wie Trifluoressigsäure oder einer Lewis-Säure wie BF₃-Etherat in der Regel bei Temperaturen zwischen etwa -40 °C und 100° in einem inerten Lösungsmittel entfernt werden.

Verbindungen der Formel 11 können vorzugsweise erhalten werden, indem man aus Verbindungen der Formel III die OR²-Gruppe mittels eines Trialkylsilans wie Triethyl- oder Triisopropylsilan und einer Lewis-Säure wie BF₃-Etherat in einem inerten Lösungsmittel wie Methylenchlorid bei Temperaturen zwischen -50°C und 50°C entfernt.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF, Wasser-Dioxan oder Kaliumcarbonat in Methanol bei Temperaturen zwischen 0 und 100° verseift werden

Silylether können z.B. mit Fluorid-haltigen Reagenzien wie HF in Pyridin oder Tetrabutylammoniumfluorid in einem inerten Lösungsmittel wie Tetrahydrofuran, oder mit anorganischen Carbonaten wie Kaliumcarbonat in einem Alkohol wie Methanol bei Temperaturen zwischen 0 und 100° gespalten werden.

### Pharmazeutische Salze und andere Formen

Die genannten Verbindungen der Formel I lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der Verbindungen der Formel I Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der Formel I der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche Verbindungen der Formel 1 hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von sauren Verbindungen der Formel I werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine Verbindung der Formel I mehr als eine Gruppe, die solche pharmazeutisch unbedenklichen Salze bilden kann, so umfaßt die Formel I auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanoli Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Ölin-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmittein gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die Wirkstoffe können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindungen enthält. Sirupe lassen sich herstellen, indem die Verbindungen in einer wäßrigen Lösung mit geeignetem Geschmack gelöst werden, während Elixiere unter Verwendung eines nichttoxischen alkoholischen Vehikels hergestellt werden. Suspensionen können durch Dispersion der Verbindungen in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie deren Salze, Tautomere und Stereoisomere, sowie die anderen Wirkstoffe lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Tautomere und Stereoisomere, sowie die anderen Wirkstoffe können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer ÖI-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I sowie des anderen Wirkstoffs hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer Verbindung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der Verbindung per se bestimmt werden.

Gegenstand der Erfindung ist ferner die Verwendung von Verbindungen der Formel I, in Kombination mit mindestens einem weiteren Arzneimittelwirkstoff, vorzugsweise zur Behandlung des Typ 1 und Typ 2 Diabetes, insbesondere zur Blutzuckersenkung.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart. Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glycogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT- 501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I ihn Kombination mit einem gemischten PPAR alpha/gamma Agonisten, wie z.B. GW 1536, AVE 8042, AVE 8134, AVE 0847, AVE 0897 oder wie in WO 00/64888, WO 00/64876, WO 03/20269 beschrieben, verabreicht.

Bei einer Ausführungsform der Erfindung werden die Verbindungen der Formel I in Kombination mit einem Fihrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht. Bei einer Ausführungsform der Erfindung werden die Verbindungen der Formel I in Kombination mit Gallensäureresorptionsinhibitor (siehe z.B. US 6,245,744 oder US 6,221, 897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht. Bei einer Ausführungsform der Erfindung werden die Verbindungen der Formel I in Kombination mit einem Lipoprotein(a) antagonist, wie z.B. Cl-1027 oder Nicotinsäure, verabreicht. Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei einer anderen Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamineregulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}-amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B.1-(4-Chloro-3-methanesulfonylmethyl-phenyl)-2-[2-(2,3-dimethyl-1H-indol-6-yloxy)ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexylethyl)-thiazol-2-ylcarbamoyl]-5,7-dimethyl-indol-1-yl}-essigsäure Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 10 00/71549), 5HT-Agonisten z.B. 1-(3-Ethyl-benzofuran-7-yl)-piperazin Oxalsäuresalz (WO 01/09111), Bombesin-Agonisten, Galanin- Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäure-tert-butylester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity, Drugs of the Future (2001), 26(9), 873-881), DA-Agonisten (Bromocriptin, Doprexin), Lipase/Amylase-Inhibitoren (z.B. WO 00140569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, daß jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 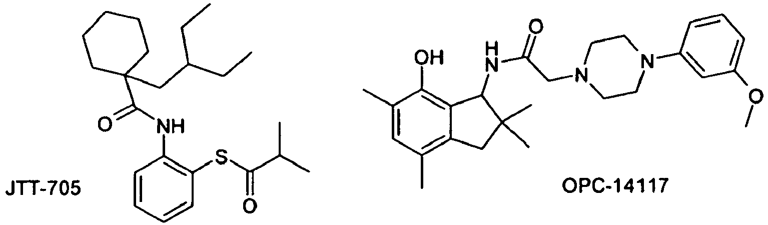 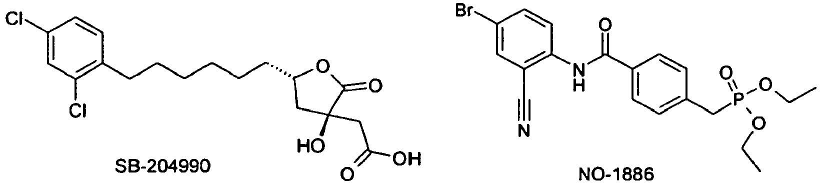 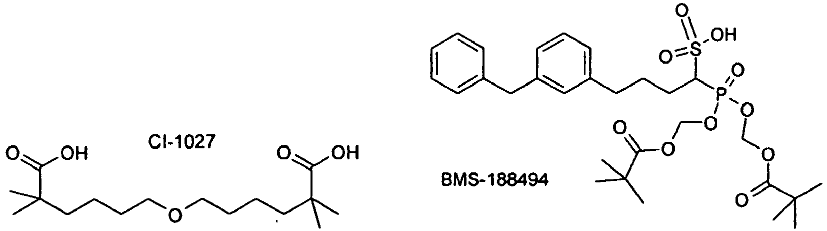 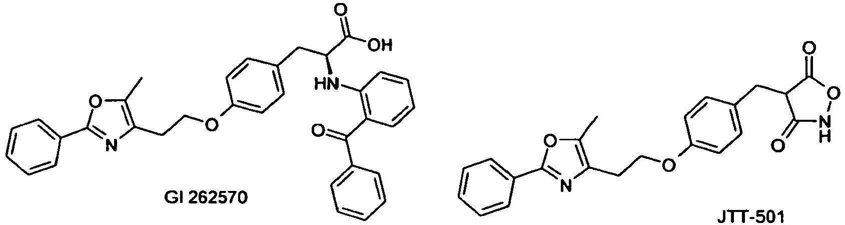 Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate, Salze und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze, Tautomere und Stereoisomere, einschließlich deren Mischungen in allen Verhältnissen,
und einer wirksamen Menge eines weiteren Arzneimittelwirkstoffs gelöst oder in lyophilisierter Form vorliegt.

Die Verbindungen können auf ihre SGLT-Hemmeigenschaften mittels BHK-Zellen getestet werden, die SGLT1 und SGLT2 exprimieren. Die Herstellung der Zellen und die Untersuchung kann wie nachstehend beschrieben durchgeführt werden.

### Konstruktion und Expression von SGLT1 in BHK-Zellen

Zur Konstruktion des SGLT1-Expressionsvektors (KL225) wurde das SLC5A1-Gen (homolog zu NM_000343) aus einer cDNA-Bank mittels Standard-PCR-Technologie amplifiziert und über NheI/XhoI-Stellen in den pcDNA3.1-Expressionsvektor (Invitrogen) kloniert, welcher Neomycin als Selektionsmarker enthielt. Bei diesem Vektor verwendet die Transkription den Enhancer/Promotor des Cytomegalievirus beim Menschen.

Der fertige Vektor KL225 wurde zusammen mit einem zusätzlichen Vektor, der ein Dihydrofolatreduktase-Gen als Selektionsmarker enthielt, in Zellen eingebracht. Die Transfektion in BHK21-Zellen (ATCC CCL-10), gezüchtet in DMEM-Medium (GIBCO/ BRL), angereichert mit 10% fötalem Kälberserum (FCS) und 20 mM Glutamin, erfolgte mit Calciumphosphat-Transfektionen gemäß Graham, F.L. und van der Ebb, A.J. (1973), Virology 52: 456 mit 5 - 20 µg ungeschnittenen Plasmiden für 10⁷ Zellen. Stabile Transfektanten wurden in Medium selektiert, das 1 mg/ml G418 (GIBCO/BRL) und 20 - 5000 nM Methotrexat als Endkonzentration enthielt, wobei nur Zellen, die das Neomycin-Gen exprimierten und das dhfr-Gen überexprimierten, wachsen konnten. Nach 2 - 3 Wochen Wachstum wurden die Zellen kloniert (0,5 Zetten/Vertiefung) und die Klone in Radioaktivitäts-Aufnahme-Tests auf SGLT-Expression untersucht.

### Konstruktion und Expression von SGLT2 in BHK-Zellen

Zur Konstruktion des SGLT2-Expressionsvektors (KL224) wurde das SLC5A2-Gen (homolog zu NM_003041) aus einer cDNA-Bank mittels Standard-PCR-Technologie amplifiziert und über NheI/XhoI-Stellen in PCIneo Expressionsvektor (Promega) kloniert, der Neomycin als Selektionsmarker enthielt. In diesem Vektor verwendet die Transkription den Enhancer/Promotor des Cytomegalie-Virus beim Menschen und das Polyadenylierungssignal von SV40.

Der fertige Vektor KL224 wurde zusammen mit einem zusätzlichen Vektor, der ein Dihydrofolatreduktase-Gen als Selektionsmarker enthielt, in Zellen eingebracht. Die Transfektion in BHK21-Zellen (ATCC CCL-10), gezüchtet in DMEM-Medium (GIBCO/ BRL), angereichert mit 10% fötalem Kälberserum (FCS) und 20 mM Glutamin, erfolgte mit Calciumphosphat-Transfektionen gemäß Graham, F.L. und van der Ebb, A.J. (1973), Virology 52: 456 mit 5 - 20 µg ungeschnittenen Plasmiden für 10⁷ Zellen. Stabile Transfektanten wurden in Medium selektiert, das 1 mg/ml G418 (GIBCO/BRL) und 20 - 5000 nM Methotrexat als Endkonzentration enthielt, wobei nur Zellen, die das Neomycin-Gen exprimierten und das dhfr-Gen überexprimierten, wachsen konnten. Nach 2 - 3 Wochen Wachstum wurden die Zellen kloniert (0,5 Zellen/Vertiefung) und die Klone in Radioaktivitäts-Aufnahme-Tests auf SGLT-Expression untersucht.

### Verfahren zur Messung der SGLT1/2-Aktivität

Prinzipiell wurde die Aufnahme von ¹⁴C-a-Methyl-D-glucopyranosid (AMG) beispielsweise in Xenopus-Oozyten, denen die entsprechende cRNA injiziert wurde, beschrieben (beispielsweise Wen-Sen Lee et al. (1994), J. Biol. Chem. 269, 12032-12039; Guofeng You et al. (1995), J. Biol. Chem. 270, 29365-29371).

Ein in 96 Vertiefungen durchgeführter Test auf Zellbasis wurde entwickelt und an die HTS-Anforderungen angepasst:

BHK-Zellen (transfiziert mit SGLT1 oder SGLT2) wurden in Mikrotiterplatten mit 96 Vertiefungen (Cultureplates, Perkin Elmer) überimpft. Nach mindestens 24 Std. wurde das Medium enternt und die Zellschicht wurde mit Testpuffer (140 mM NaCl, 2 mM KCI, 1 mM CaCl₂, 1 mM MgCl₂, 10 mM HEPES, 5 mM Tris, mit 1 M KOH auf pH-Wert 7,4 eingestellt) gewaschen. Nach Zugabe von 40 µl Testpuffer 50 µl AMG (50 µM für SGLT1 bzw. 2 mM für SGLT2) in Gegenwart oder Abwesenheit von Verbindungen wurden die Zellen in einem Gesamtvolumen von 100 µl bei 37°C für 90 min. inkubiert. Der Überstand wurde abgesaugt und verworfen. Die Zellen wurden gewaschen und durch Zugabe von 50 µl Wasser lysiert. Nach 10 min bei Raumtemperatur wurden 200 µl Micrsoscint 40 (Perkin Elmer) hinzu gefügt. Die Radioaktivität wurde in einem Topcount Mikroplatten-Szintillationszähler (Perkin Elmer) gezählt. Die unspezifische Aufnahme wurde in natriumfreiem Testpuffer (266 mM Saccharose, 2 mM KCI, 1 mM CaCl₂, 1 mM MgCl₂, 10 mM HEPES, 5 mM Tris, mit 1 M KOH auf pH-Wert 7,4 eingestellt) bestimmt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): EI (Elektronenstoß-Ionisation) M⁺
FAB (Fast Atom Bombardment) (M+H)⁺
ESI (Electrospray lonization) (M+H)⁺ (wenn nichts anderes angegeben)

### LC-MS und HPLC-Bedingungen

Die in den nachfolgenden Beispielen angegebenen M+H⁺-Daten sind die Messergebnisse der LC-MS-Messungen:
Hewlett Packard System der HP 1100 Serie mit den folgenden Merkmalen:
Ionenquelle: Electrospray (positive mode); Scan:100-1000 m/z;
Fragmentier-Spannung : 60 V ; Gas-Temperatur :300 °C, DAD :220 nm.

Flussrate: 2.4 ml/Min. Der verwendete Splitter reduzierte nach dem DAD die Flussrate für das MS auf 0,75 ml/Min.
Säule: Chromolith SpeedROD RP-18e 50-4.6
Lösungsmittel: Lichrosolv-Qualität der Fa. Merck KGaA
Lösungsmittel A: H2O (0.01 % TFA)
Lösungsmittel B: ACN (0.008% TFA)

### Beispiel 1

Die Herstellung von 6-(β-D-glucopyranos-1-yl)-1-(4-methoxy-phenyl)-indan ("A1") erfolgt analog nachstehendem Schema: 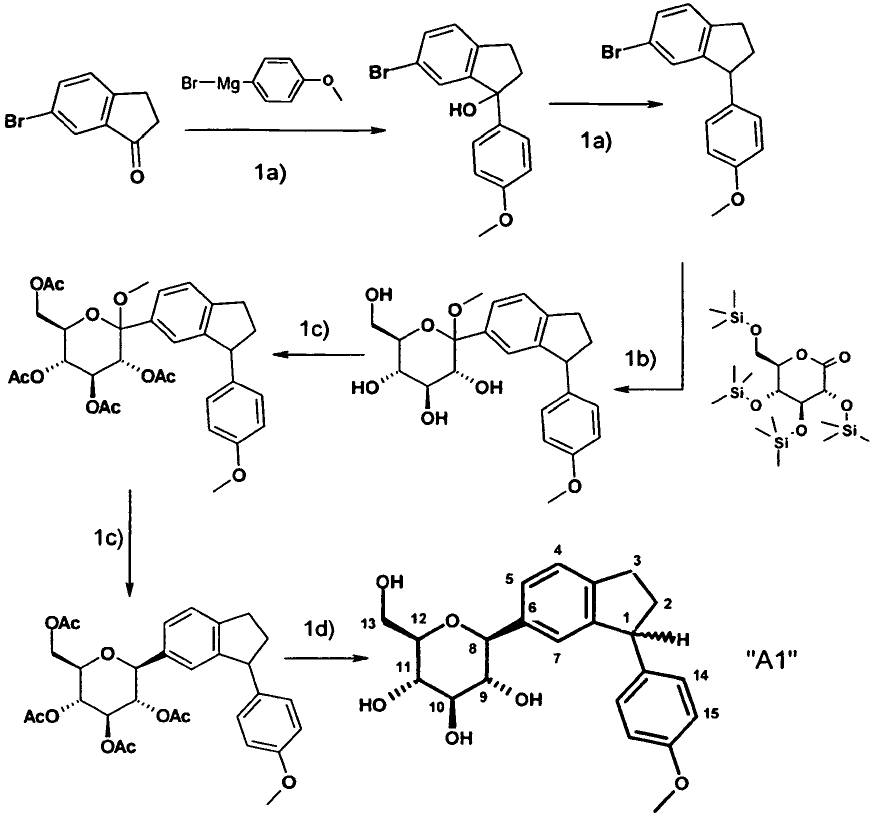
1a) Eine Lösung von 0.9 g (4.26 mmol) 6-Brom-1-indanon in 10 ml absolutem Tetrahydrofuran wird unter Stickstoff bei -20 °C tropfenweise mit 6.4 ml (6.4 mmol) einer Lösung von 4-Methoxyphenylmagnesiumbromid in Tetrahydrofuran (1 M Lsg) versetzt. Anschließend wird die Reaktionslösung innerhalb von 12 Stunden auf Raumtemperatur erwärmt, dann mit 45 ml 10%iger Ammoniumchlorid-Lösung versetzt und schließlich zweimal mit je 20 ml Methylenchlorid extrahiert. Nach dem Trocknen der vereinigten organischen Phasen über Natriumsulfat und Abziehen des Lösungsmittels wird das Rohprodukt säulechromatographisch an Kieselgel (Petrolether/Essigsäureethylester 4:1) gereinigt. Man erhält so 1.1 g 6-Brom-1-(4-methoxy-phenyl)-indan-1-ol als leichtgelbes Öl. LC/MS : 302 (M+H-18 (Wasser)). Dieses Öl wird nun in 20 ml Methylenchlorid gelöst, die Lösung auf -20 °C gekühlt und nach Zugabe von 0.8 ml (5 mmol) Triethylsilan mit 0.53 ml (4.2 mmol) Bortrifluorid-Diethylether-Komplex versetzt. Anschließend wird die Reaktionslösung auf Raumtemperatur erwärmt, vier Stunden bei Raumtemperatur gerührt und dann nacheinander mit 20 ml gesättigter Natriumhydrogencarbonat- und 20 ml gesättigter Natriumchlorid-Lösung gewaschen. Nach dem Trocknen der Methylenchloridlösung über Natriumsulfat und Abziehen des Lösungsmittels wird das Rohprodukt säulechromatographisch an Kieselgel (Petrolether / Essigsäureethylester 95:5) gereinigt. Man erhält so 0.52 g 6-Brom-1-(4-methoxy-phenyl)-indan als farbloses Öl; ¹H-NMR (d₆-DMSO): δ 7.34 ppm (d, 1H, J=7.9 Hz), 7.26 ppm (d, 1H, J=7.9 Hz), 7.11 ppm (d, 2H, J=8.97 Hz), 6.93 ppm (s, 1H), 6.90 ppm (d, 2H, J=8.97 Hz), 4.31 ppm (t, 1H, J=8.27 Hz), 3.74 ppm (s, 3H, OCH3), 2.98 ppm (m, 1H), 2.85 ppm (m, 1H), 2.48 ppm (m, 1H), 2.0 ppm (m, 1H).
1b) Eine Lösung von 0.53 g (1.75 mmol) 6-Brom-1-(4-methoxyphenyl)-indan in 15 ml trockenem Diethylether wird unter Stickstoff bei -78 °C mit 2.05 ml tert.-Butyllithium (1.7 M in Pentan) versetzt und zwei Stunden bei dieser Temperatur gerührt. Zu dieser Lösung tropft man nun eine Lösung von 0.9 g (1.9 mmol) 2,3,4,6-Tetrakis-O-(trimethylsilyl)-D-glucopyranon in 5 ml Diethylether bei -78 °C, rührt anschließend die Reaktionsmischung noch drei Stunden bei -78 °C, fügt dann eine Lösung von 0.41 ml Methansulfonsäure in 5 ml Methanol hinzu und erwärt schließlich die Reaktionsmischung innerhalb von 12 Stunden auf Raumtemperatur. Nach Zugabe von 10 ml einer 10%iger Natriumhydrogencarbonatlösung unter Eiskühlung und Phasentrennung wird die wässrige Phase zweimal mit je 20 ml Essigsäureethylester extrahiert. Nach dem Trocknen der vereinigten organischen Phasen über Natriumsulfat und Abziehen des Lösungsmittels wird das Rohprodukt säulechromatographisch an Kieselgel (Essigsäureethylester) gereinigt. Man erhält so 0.25 g 6-(1-Methoxy-D-glucopyranos-1-yl)-1-(4-methoxy-phenyl)-indan als fabloses Öl. LC/MS: 439 (M+Na).
1c) Eine Lösung von 0.22 g (0.53 mmol) 6-(1-Methoxy-D-glucopyranos-1-yl)-1-(4-methoxy-phenyl)-indan, 0.66 ml N-Ethyldiisopropylamin und 6.5 mg 4-(Dimethylamino)-pyridin in 15 ml Tetrahydrofuran wird bei -5 °C mit 0.32 ml (3.4 mmol) Essigsäureanhydrid versetzt. Man lässt nun die Reaktionsmischung auf Raumtemperatur kommen, rührt noch weitere 12 Stunden, zieht dann das Lösungsmittel im Vakuum ab, nimmt den Rückstand in 10 ml Essigsäureethylester auf, wäscht die entstandene Lösung mit je 10 ml Wasser und gesättigter Natriumchloridlösung und trocknet die organische Phase über Natriumsulfat. Nach dem Abziehen des Lösungsmittels erhält man 0.32 g 6-(1-Methoxy-2,3,4,6-Tetra-O-acetyl-D-gluco-pyranos-1-yl)-1-(4-methoxy-phenyl)-indan als gelbliches Öl. LC/MS: 607 (M+Na). Dieses Öl wird nun in 20 ml Methylenchlorid gelöst, die Lösung auf -40 °C gekühlt und nach Zugabe von 0.29 ml Triethylsilan und 10 µl Wasser mit 0.15 ml Bortrifluorid-Diethylether-Komplex versetzt. Anschließend lässt man die Reaktionslösung noch zwei Stunden bei -30 °C rühren, erwärmt sie dann auf Raumtemperatur und rührt noch zwölf Stunden weiter. Anschließend wäscht man die Methylenchloridlösung mit je 10 ml Wasser und gesättigter Natriumchloridlösung, trocknet die organische Phase über Natriumsulfat, engt dann die Methylenchloridlösung zu Trockne ein und reinigt das Rohprodukt säulechromatographisch an Kieselgel (Petrolether/Essigsäureethylester 7:3). Man erhält 0.23 g 6-(2,3,4,6-Tetra-O-acetyl-β-D-glucopyranos-1-yl)-1-(4-methoxyphenyl)-indan als 1/1-Epimerengemisch an C-1; farbloses Öl; LC/MS: 555 (M+H).
1d) Eine Lösung von 80 mg (0.144 mmol) 6-(2,3,4,6-Tetra-O-acetyl-β-D-glucopyranos-1-yl)-1-(4-methoxy-phenyl)-indan, 4mg Lithiumhydroxyd in 2 ml Wasser, 6 ml Methanol und 6 ml Tetrahydrofuran wird 12 Stunden bei Raumtemperatur gerührt. Anschließend engt man die Reaktionslösung zur Trockne ein, nimmt den Rückstand in 5 ml 10%iger Kaliumhydrogensulfat Lösung auf und extrahiert die wässrige Lösung dreimal mit je 10 ml Essigsäureethylester. Nach dem Trocknen der vereinigten organischen Phasen über Natriumsulfat und Abziehen des Lösungsmittels wird das Produkt mit Diethylether/Petrolether kristallisiert. Man erhält 34 mg "A1" als 1/1-Epimerengemisch an C-1; farblose Kristalle; LC/MS: 409 (M+Na); ¹H-NMR (CH₃OD): δ 7.24 ppm (m, 2H; H-4, H-5), 7.08 ppm (d, 2H; H-14), 6,96 und 6.93 ppm (2 s, 1H; H-7, Epimer-1 und Epimer-2), 6,84 (dd, 2H; H-15), 4.27 ppm (q, 1H; H-1), 4.05 ppm (t, 1H; H-8), 3.86 (d, 1H; H-13), 3.76 ppm (s, 3H; OCH₃), 3.65 ppm (m, 1H; H-13), 3.43 ppm (m, 1H; H-10), 3.35 ppm (m, 2H; H-11, und H-12), 3.30 ppm (m, 1H; H-9), 3.00 ppm (m, 1H; H-3), 2.90 ppm (m, 1H; H-3), 2.55 ppm (m, 1H; H-2), 1.95 ppm (m, 1H; H-2).

### Beispiel 2

### Die Herstellung von 6-(β-D-glucopyranos-1-yl)-1S-(4-methoxy-phenyl)-indan ("A2") und von 6-(β-D-glucopyranos-1-yl)-1R-(4-methoxy-phenyl)-indan ("A3")

Die säulenchromatographische Trennung von 110 mg des 1/1-Epimerengemisches 1c) an chiraler Phase liefert die beiden optisch aktiven Tetraacetate 6-(2,3,4,6-Tetra-O-acetyl-β-D-glucopyranos-1-yl)-1*S*-(4-methoxyphenyl)-indan und 6-(2,3,4,6-Tetra-O-acetyl-β-D-glucopyranos-1-yl)-1*R*-(4-methoxy-phenyl)-indan, deren Hydrolyse analog 1d) 35 mg 6-(β-D-glucopyranos-1-yl)-1 S-(4-methoxy-phenyl)-indan ("A2") (LC/MS: 409 (M+Na)) und 55 mg 6-(β-D-glucopyranos-1-yl)-1*R*-(4-methoxy-phenyl)-indan ("A3") (LC/MS: 409 (M+Na)) ergibt.

### Beispiel 3

### 6-(β-D-glucopyranos-1-yl)-1-(4-ethoxy-phenyl)-indan ("A4")

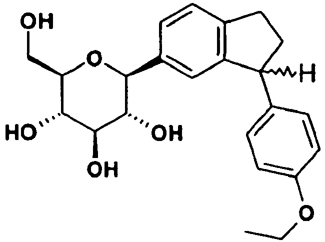

Analog zu 1a)-1d) erhält man "A4"; LC/MS: 423 (M+Na).

### Beispiel 4

### 7-(β-D-glucopyranos-1-yl)-1-(4-methoxy-phenyl)-1,2,3,4-tetrahydronaphthalin ("A5")

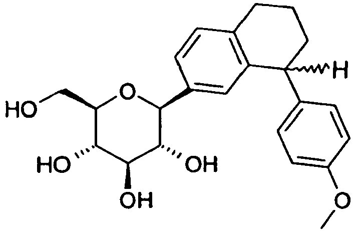

Ausgehend von 7-Brom-3,4-dihydro-2H-naphthalin-1-on erhält man analog zum Beispiel 1a)-1d) "A5" als weißes Pulver; LC/MS: 423 (M+Na).

### Beispiel 5

### 7-(1-Methoxy-D-glucopyranos-1-yl)-1-(4-methoxy-phenyl)-3,4-dihydronaphthalin ("A6")

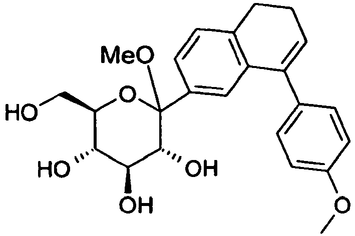

5a) Eine Lösung aus 0.55 g (1.65 mmol) des analog zum Beispiel 1a) aus 7-Brom-3,4-dihydro-2*H*-naphthalin-1-on und 4-methoxyphenylmagnesiumbromid erhaltenen 7-Brom-1-(4-methoxy-phenyl)-1,2,3,4-tetrahydro-naphthalin-1-ol und 0.2 ml Trifluoressigsäure in 20 ml Tetrahydrofuran wird 12 Stunden bei 50 °C erwärmt und anschließend zur Trockne eingedampft. Der Rückstand wird mit 10 ml gesättigter Natriumhydrogencarbonat versetzt und die wässrige Mischung zweimal mit je 10 ml Essigsäureethylester extrahiert. Nach dem Trocknen der vereinigten organischen Phasen über Natriumsulfat und Abziehen des Lösungsmittels wird das Rohprodukt säulechromatographisch an Kieselgel (Petrolether Essigsäureethylester 9:1) gereinigt. Man erhält 0.54 g 7-Brom-1-(4-methoxy-phenyl)-3,4-dihydro-naphthalin; LC/MS: 338 (M+Na).
5b) Aus 7-Brom-1-(4-methoxy-phenyl)-3,4-dihydro-naphthalin und 2,3,4,6-Tetrakis-O-(trimethylsilyl)-D-glucopyranon erhält man analog zu Beispiel 1b) die Verbindung "A6" als gelbliches Harz; LC/MS: 451 (M+Na).

### Beispiel 6

### 5-(β-D-glucopyranos-1-yl)-3-(4-methoxy-phenyl)-benzofuran ("A7") und 5-(β-D-glucopyranos-1-yl)-2-(4-methoxy-phenyl)-benzofuran ("A8")

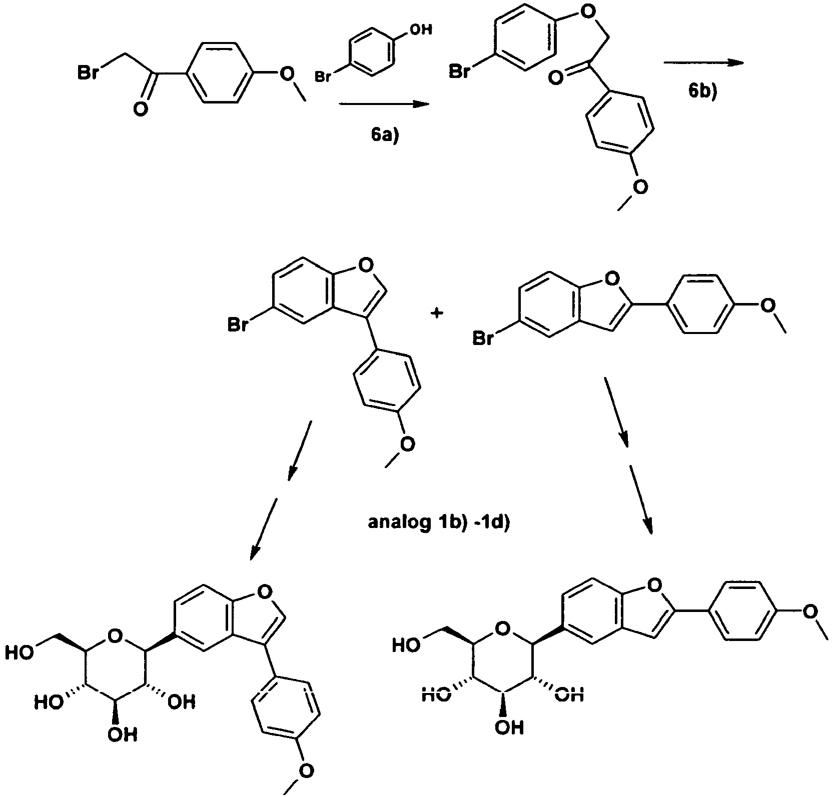

6a) Eine Lösung von 20 g (0.115 Mol) 4-Bromphenol und 26.5 g (0.115 Mol) 2-Brom-4'-methoxyacetophenon in 400 Acetonitril wird mit 24 g Kaliumcarbonat versetzt und die Reaktionsmischung fünf Stunden am Rückfluss erhitzt. Anschließend wird der Niederschlag abfiltriert, das Filtrat zur Trockne eingedampft, der Rückstand in 10 ml Wasser aufgenommen und die wässrige Lösung zweimal mit je 10 ml Essigsäureethylester extrahiert. Die vereinigten organischen Phasen werden mit 10 ml gesättigter Natriumchlorid-Lösung gewaschen und über Natriumsulfat getrocknet. Nach Abziehen des Lösungsmittels wird der Rückstand mit 50 ml Diethylether verrieben und das dabei entstehende kristalline Produkt unter Vakuum abgesaugt. Man erhält 35.5 g 2-(4-Brom-phenoxy)-1-(4-methoxy-phenyl)-ethanon als beigen Feststoff.
6b) Eine Mischung aus 35.5 g (0.11 mol) 2-(4-Brom-phenoxy)-1-(4-methoxy-phenyl)-ethanon 6a) und 75 g Polyphosphorsäure in 900 ml Xylol wird 15 Stunden bei 160 °C erhitzt, dann auf Raumtemperatur abgekühlt, die Xylollösung vom zähflüssigen Bodensatz abdekantiert und der Kolbenbodensatz noch zweimal mit je 50 ml Xylol gewaschen. Die vereinigten Xylolphasen werden hintereinander mit je 50 ml gesättigter Natriumchlorid-Lösung und Wasser gewaschen und über Natriumsulfat getrocknet. Nun reduziert man die Xylolmenge im Vakuum auf ca. 100 ml und filtriert das ausgefallene 5-Brom-2-(4-methoxy-phenyl)-benzofuran (6b)/2) (5g farblose Kristalle; F.188.6 °C) ab. Das Filtrat wird dann zur Trockne eingeengt, der zähflüssige Rückstand, der allmählich kristallisiert, mit 10 ml Methanol verrieben und das entstandene Kristallisat unter Vakuum abgesaugt. Man erhält 19.2 g 5-Brom-3-(4-methoxy-phenyl)-benzofuran (6b)/1) als gelblichen Feststoff (F. 86.8 °C); ¹H-NMR (d₆-DMSO):
   **6b)/1**: 8.35 ppm (s, 1H), 8.0 ppm (s, 1H), 7.65 (dd, 3H), 7.55 (d, 1H), 7.06 ppm (d, 2H), 3.84 ppm (s, 3H);
   **6b)/2**: 7.87 ppm (d, 2H), 7.82 ppm (s, 1H), 7.57 ppm (d, 1H), 7.45 ppm (d, 1H), 7.25 ppm (s, 1H), 7.06 ppm (d, 2H), 3.85 ppm (s, 3H).
6c) Aus 5-Brom-3-(4-methoxy-phenyl)-benzofuran 6b)/1 und 2,3,4,6-Tetrakis-O-(trimethylsilyl)-D-glucopyranon erhält man analog zu Beispiel 1b)-1d) die Verbindung "A7" als braunen Feststoff; LC/MS: 387 (M+H); ¹H-NMR (d₆-DMSO): δ 8.24 ppm (s, 1H), 7.78 ppm (s, 1H), 7.65 (dd, 3H), 7.55 (d, 1H), 7.06 ppm (d, 2H), 4.93 ppm (breites s, 2H; 2xOH), 4.73 (d, 1H; OH), 4.45 ppm (breites s, 1H; OH), 4.25 ppm (d, 1H), 3.80 ppm (s, 3H), 3.70 ppm (breites d, 1H), 3.48 ppm (m, 1H), 3.3 ppm (m, 3H).
6d) Aus 5-Brom-2-(4-methoxy-phenyl)-benzofuran 6b)/2 und 2,3,4,6-Tetrakis-O-(trimethylsilyl)-D-glucopyranon erhält man analog zu Beispiel 1b)-1d) die Verbindung "A8" als farblose Kristalle; LC/MS: 387 (M+H); ¹H-NMR (d₆-DMSO): δ 7.92 ppm (d, 2H), 7.65 ppm (s, 1H), 7.52 ppm (d, 1H), 7.35 ppm (d, 1H), 7.31 ppm (s, 1H), 7.12 ppm (d, 2H), 4.98 ppm (breites s, 2H; 2xOH), 4.80 (d, 1H; OH), 4.50 ppm (t, 1H; OH), 4.17 ppm (d, 1H), 3.90 ppm (s, 3H), 3.70 ppm (dd, 1H), 3.55 ppm (m, 1H), 3.32 ppm (m, 3H).

### Beispiel 7

### 5-(β-D-glucopyranos-1-yl)-3-(4-methoxy-phenyl)-2,3-dihydro-benzofuran ("A9")

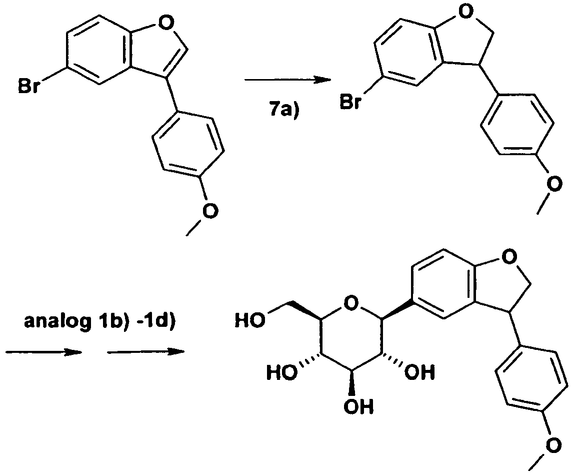

7a) Eine Suspension aus 5.0 g (16.5 mmol) 5-Brom-3-(4-methoxyphenyl)-benzofuran 6b)/1 und 100 mlTrifluoressigsäure wird mit 10.5 ml Triethylsilan versetzt, wobei man allmählich eine homogene Lösung erhält. Die Reaktionsmischung wird nun 30 Stunden bei Raumtemperatur gerührt, dann im Vakuum eingeengt, der Rückstand in 30 ml Wasser aufgenommen und dreimal mit je 30 ml Essigsäureethylester extrahiert. Die vereinigten organischen Phasen werden nacheinander mit je 10 ml 10%iger Natriumhydrogencarbonatlösung, gesättigter Natriumchlorid-lösung und Wasser gewaschen und über Natriumsulfat getrocknet. Nach Abziehen des Lösungsmittels am Rotationsverdampfer wird das Rohprodukt säulechromatographisch an Kieselgel (n-Heptan / Essigsäureethylester 95:5) gereinigt. Man erhält 4.3 g 5-Brom-3-(4-methoxy-phenyl)-2,3-dihydro-benzofuran als farblose Kristalle; LC/MS: 306 (M+H);
   ¹H-NMR (d₆-DMSO): δ 7.30 ppm (d mit Feinaufspaltung, 1H), 7.14 ppm (d, 2H), 7.08 ppm (s mit Feinaufspaltung, 1H), 6.90 ppm (d, 2H), 6.85 ppm (d, 1H), 4.90 ppm (t, 1H), 4.74 ppm (dd, 1H), 4.40 ppm (dd, 1H), 3.73 ppm (s, 3H).
7b) Aus 5-Brom-3-(4-methoxy-phenyl)-2,3-dihydro-benzofuran 7a) und 2,3,4,6-Tetrakis-O-(trimethylsilyl)-D-glucopyranon erhält man analog zu Beispiel 1b)-1d) die Verbindung "A9" als braunen Feststoff; LC/MS: 389 (M+H); ¹H-NMR (CD₃OD): δ 7.23 ppm (d, 1H), 7.15 ppm (d, 2H), 7.08 und 7.05 ppm (2s, 1H; Epimer-1 und Epimer-2), 6.90 ppm (d, 2H), 6.81 ppm (d, 1H), 4.87 ppm (t, 1H), 4.65 ppm (t, 1H), 4.35 ppm (t, 1H), 4.04 ppm (d, 1H), 3.86 ppm (d, 1H), 3.79 ppm (s, 3HL; OCH₃), 3.68 ppm (dd, 1H), 3.44 ppm (t, 1H), 3.36 ppm (m, 3H), 3.07 ppm (t, 1H).

### Beispiel 8

### 5-(β-D-glucopyranos-1-yl)-3-(4-ethoxy-phenyl)-benzofuran ("A10") und 5-(β-D-glucopyranos-1-yl)-3-(4-ethoxy-phenyl)-2,3-dihydro-benzofuran ("A11")

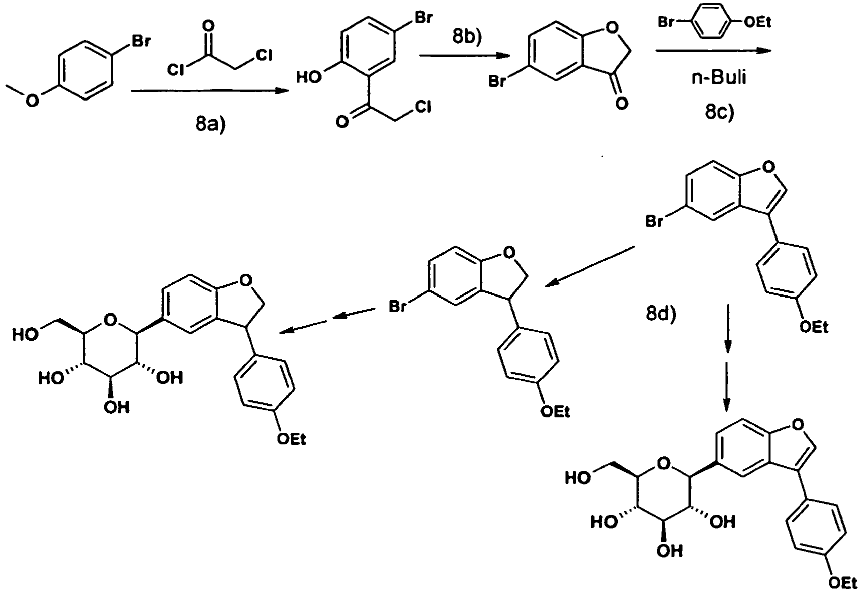

8a) Eine Lösung von 10 ml (80.2 mmol) 4-Bromanisol und 18.5 ml (232.6 mmol) Chloracetylchlorid in 90 ml Methylenchlorid wird bei einer Temperatur kleiner 30 °C mit 35.3 g (264.6 mmol) Aluminiumtrichlorid portionsweise versetzt und die entstandene Reaktionsmischung sechs Stunden am Rückfluss erhitzt. Anschließend fügt man der Reaktionsmischung unter Eiskühlung 100 ml Eiswasser vorsichtig hinzu, trennt die organische Phase, wäscht die wässrige Phase zweimal mit je 50 ml Methylenchlorid, wäscht dann die vereinigten organischen Phasen mit 50 ml Wasser und trocknet sie über Natriumsulfat. Nach Abfiltrieren des Trockenmittels und Abziehen des Lösungsmittels erhält man 11.7 g 1-(5-Brom-2-hydroxy-phenyl)-2-chlor-ethanon als beigen Feststoff; F. 79 °C (Lit. F. 80-82 °C).
8b) Eine Lösung aus 8.34 g (33.4 mmol) 1-(5-Brom-2-hydroxy-phenyl)-2-chlor-ethanon und 3.3 g (40.1 mmol) wasserfreiem Natriumacetat in 350 ml Methanol wird eine Stunde bei 65 °C erhitzt, anschließend zur Trockne eingeengt, der Rückstand in 50 ml Wasser aufgenommen und die wässrige Mischung zweimal mit je 50 ml Methylenchlorid extrahiert. Nach dem Trocknen der vereinigten organischen Phasen über Natriumsulfat und Abziehen des Lösungsmittels wird das Rohprodukt säulechromatographisch an Kieselgel (n-Heptan / Methylenchlorid 7:3) gereinigt. Man erhält 2.14 g 5-Brom-benzofuran-3-on als gelben Feststoff; LC/MS: 214 (M+H).
8c) Eine auf -78 °C gekühlte Lösung von 1.03 ml (7.23 mmol) 4-Bromphenetol in 35 ml Tetrahydrofuran wird unter Stickstoff mit 4.95 ml (15%ige Lösung in n-Hexan) n-Butyllithium versetzt und eine Stunde bei -78 °C gerührt. Anschließend tropft man eine Lösung von 1.4 g (6.6 mmol) 5-Brom-benzofuran-3-on so zu, dass die Temperatur nicht über -70 °C ansteigt, rührt eine Stunde bei -78 °C weiter, lässt dann die Reaktionsmischung auf Raumtemperatur kommen rührt noch 15 Stunden bei Raumtemperatur, versetzt sie schließlich mit 10 ml 1N Salzsäure und rührt noch eine Stunde weiter. Nach Zugabe von 20 ml Wasser wird das Tetrahydrofuran im Vakuum abgezogen und die verbleibende wässrige Mischung dreimal mit je 10 ml Diethylether extrahiert. Nach dem Waschen der vereinigten organischen Phasen mit 10 ml gesättigter Natriumchlorid-lösung und Trocknen über Natriumsulfat wird das Lösungsmittel am Rotationsverdampfer abgezogen und das Rohprodukt säulechromatographisch an Kieselgel (Petrolether / Essigsäureethylester 98:2) gereinigt. Man erhält 0.95 g 5-Brom-3-(4-ethoxy-phenyl)-benzofuran als farbloses Öl; LC/MS: 318 (M+H);
   ¹H-NMR (d₆-DMSO): δ 8.34 ppm (s, 1H), 8.00 ppm (s mit Feinaufspaltung, 1H), 7.66 ppm (d, 1H), 6.63 ppm (d, 2H), 7.53 ppm (d mit Feinaufspaltung, 1H), 7.05 ppm (d, 2H), 4.07 ppm (q, 2H), 1.36 ppm (t, 3H).
8d) Analog zu Beispiel 7a) erhält man aus 5-Brom-3-(4-ethoxyphenyl)-benzofuran, Trifluoressigsäure und Triethylsilan 5-Brom-3-(4-ethoxy-phenyl)-2,3-dihydro-benzofuran als farbloses Öl; LC/MS: 320 (M+H); ¹H-NMR (d₆-DMSO): δ 7.30 ppm (d mit Feinaufspaltung, 1H), 7.12 ppm (d, 2H), 7.09 ppm (s mit Feinaufspaltung, 1H), 6.89 ppm (d, 2H), 6.85 ppm (d, 1H), 4.89 ppm (t, 1H), 4.70 ppm (dd, 1H), 4.39 ppm (dd, 1H), 3.99 ppm (q, 2H), 1.32 ppm (t, 3H).
8e) Aus 5-Brom-3-(4-ethoxy-phenyl)-benzofuran 8c) und 2,3,4,6-Tetrakis-O-(trimethylsilyl)-D-glucopyranon erhält man analog zu Beispiel 1b)-1d) die Verbindung "A10" als weißen Feststoff; LC/MS: 401 (M+H).
8f) Aus 5-Brom-3-(4-ethoxy-phenyl)-2,3-dihydro-benzofuran 8d) und 2,3,4,6-Tetrakis-O-(trimethylsilyl)-D-glucopyranon erhält man analog zu Beispiel 1b)-1d) die Verbindung "A11" als hellbraunen Feststoff; LC/MS: 403 (M+H).

### Beispiel 10

### Die Herstellung von 6-(β-D-glucopyranos-1-yl)-3S-(4-methoxy-phenyl)-indan ("A13") und von 6-(β-D-glucopyranos-1-yl)-3R-(4-methoxy-phenyl)-indan ("A14")

Die säulenchromatographische Trennung von 700 mg des 1/1-Epimerengemisches 7b) ("A9") an chiraler Phase liefert 270 mg 6-(β-D-glucopyranos-1-yl)-3S-(4-methoxy-phenyl)-indan ("A13") und 134 mg (LC/MS: 411 (M+Na)) und 6-(β-D-glucopyranos-1-yl)-3R-(4-methoxyphenyl)-indan ("A14"), (LC/MS: 411 (M+Na)).

### Pharmakologische Daten

Affinität zu Rezeptoren

**Tabelle 1**

| Verbindung Nr. | SGLT₁-IC₅₀ | SGLT₂-IC₅₀ |
|---|---|---|
| "A1" | C | A |
| "A2" | C | A |
| "A3" | C | C |
| "A4" | C | A |
| "A5" | C | A |
| "A6" | C | C |
| "A7" | C | B |
| "A8" | C | C |
| "A9" | C | A |
| "A12" | C | C |
| "A13" | C | A |
| "A14" | C | C |

IC₅₀: 10 nM - 1µM = A
1 µM-10 µM = B
>10 µM = C

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 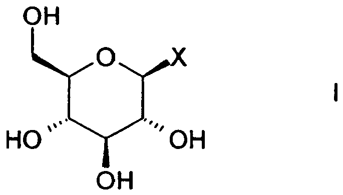 worin
X 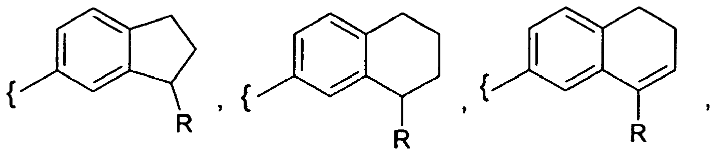 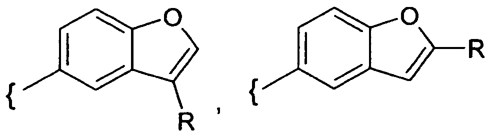 oder 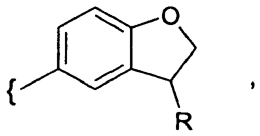 wobei der 6-gliedrige aromatische Ring ein- oder zweifach durch Hal, OR⁵, NR⁵R⁶, CN, COOR⁵, CONR⁵R⁶, -OCOA, NR⁵COR⁶ und/oder NR⁵SO₂A substituiert sein kann,
R Carb, Ar oder Het,
R⁵, R⁶ jeweils unabhängig voneinander H oder A,
Carb Cycloalkyl mit 3-7 C-Atomen,
Ar unsubstituiertes oder ein-, zwei-, drei-, vier- oder fünffach durch Hal, A, Benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)pA und/oder -(CR⁵R⁶)ₘ-COOR⁵ substituiertes Phenyl, Naphthyl oder Biphenyl,
Het einen ein- oder zweikernigen gesättigten, ungesättigten oder aromatischen Heterocyclus mit 1 bis 4 N-, O- und/oder S-Atomen, der ein-, zwei- oder dreifach durch Hal, A, Benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)ₚA, -(CR⁵R⁶)ₘ-COOR⁵, =S, =NR¹ und/oder =O (Carbonylsauerstoff) substituiert sein kann,
A unverzweigtes oder verzweigtes Alkyl mit 1-10 C-Atomen, worin eine, zwei oder drei CH₂-Gruppen unabhängig voneinander durch O, S, SO, SO₂, NR⁵, -C≡C- und/oder durch -CH=CH-Gruppen und/oder worin auch 1-7 H-Atome durch F und/oder Cl ersetzt sein können, oder Cycloalkyl mit 3-7 C-Atomen,
Hal F, Cl, Br oder I,
m 0, 1, 2 oder 3,
p 0, 1 oder 2,
bedeuten,
sowie ihre pharmazeutisch verwendbaren Salze, Tautomere und Stereoisomere, einschließlich deren Mischungen in allen Verhältnissen.

2. Verbindungen nach Anspruch 1, worin
worin
R⁵, R⁶ jeweils unabhängig voneinander H oder Methyl,
bedeuten,
sowie ihre pharmazeutisch verwendbaren Salze, Tautomere und Stereoisomere, einschließlich deren Mischungen in allen Verhältnissen.

3. Verbindungen nach Anspruch 1 oder 2, worin
R Ar bedeutet,
sowie ihre pharmazeutisch verwendbaren Salze, Tautomere und
Stereoisomere, einschließlich deren Mischungen in allen Verhältnissen.

4. Verbindungen gemäß einem oder mehreren der Ansprüche 1-3, worin
Ar unsubstituiertes oder ein-, zwei- oder dreifach durch Hal, A und/oder OR⁵ substituiertes Phenyl,
bedeutet,
sowie ihre pharmazeutisch verwendbaren Salze, Tautomere und Stereoisomere, einschließlich deren Mischungen in allen Verhältnissen.

5. Verbindungen gemäß einem oder mehreren der Ansprüche 1-4, worin
A unverzweigtes oder verzweigtes Alkyl mit 1-6 C-Atomen, worin 1-7 H-Atome durch F und/oder Cl ersetzt sein können,
bedeutet,
sowie ihre pharmazeutisch verwendbaren Salze, Tautomere und Stereoisomere, einschließlich deren Mischungen in allen Verhältnissen.

6. Verbindungen nach Anspruch 1,
worin
X 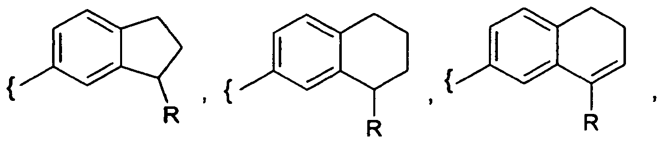 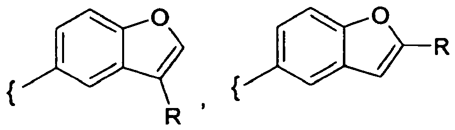 oder 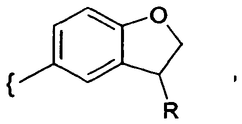
R Ar,
R⁵ H oder A,
Ar unsubstituiertes oder ein-, zwei- oder dreifach durch Hal, A und/oder OR⁵ substituiertes Phenyl,
A unverzweigtes oder verzweigtes Alkyl mit 1-6 C-Atomen, worin 1-7 H-Atome durch F und/oder Cl ersetzt sein können,
Hal F, Cl, Br oder I
bedeuten,
sowie ihre pharmazeutisch verwendbaren Salze, Tautomere und Stereoisomere, einschließlich deren Mischungen in allen Verhältnissen.

7. Verbindungen nach Anspruch 1 ausgewählt aus der Gruppe
| Nr. | Name und/oder Struktur |
|---|---|
| "A1" | 6-(β-D-glucopyranos-1-yl)-1-(4-methoxy-phenyl)-indan |
| "A2" | 6-(β-D-glucopyranos-1-yl)-1S-(4-methoxy-phenyl)-indan |
| "A3" | 6-(β-D-glucopyranos-1-yl)-1R-(4-methoxy-phenyl)-indan |
| "A4" | 6-(β-D-glucopyranos-1-yl)-1-(4-ethoxy-phenyl)-indan |
| "A5" | 7-(β-D-glucopyranos-1-yl)-1-(4-methoxy-phenyl)-1,2,3,4-tetrahydro-naphthalin |
| "A7" | 5-(β-D-glucopyranos-1-yl)-3-(4-methoxy-phenyl)-benzofuran |
| "A8" | 5-(β-D-glucopyranos-1-yl)-2-(4-methoxy-phenyl)-benzofuran |
| "A9" | 5-(β-D-glucopyranos-1-yl)-3-(4-methoxy-phenyl)-2,3-dihydro-benzofuran |
| "A10" | 5-(β-D-glucopyranos-1-yl)-3-(4-ethoxy-phenyl)-benzofuran |
| "A11" | 5-(β-D-glucopyranos-1-yl)-3-(4-ethoxy-phenyl)-2,3-dihydro-benzofuran |
sowie ihre pharmazeutisch verwendbaren Salze, Tautomere und Stereoisomere, einschließlich deren Mischungen in allen Verhältnissen.

8. Verfahren zur Herstellung von Verbindungen der Formel I nach den Ansprüchen 1-7 sowie ihrer pharmazeutisch verwendbaren Salze, Tautomere und Stereoisomere, **dadurch gekennzeichnet, daß** man aus einer Verbindung der Formel II 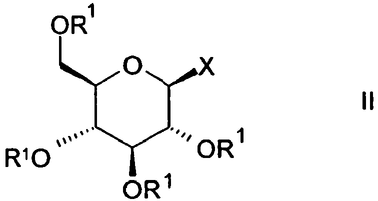 worin
X die in Anspruch 1 angegebene Bedeutung hat, und R¹ eine Hydroxyschutzgruppe bedeutet,
R¹ abspaltet,
und/oder
eine Base oder Säure der Formel I in eines ihrer Salze umwandelt.

9. Arzneimittel, enthaltend mindestens eine Verbindung der Formel I nach einem oder mehreren der Ansprüche 1 bis 7 und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

10. Arzneimittel enthaltend mindestens eine Verbindung der Formel I gemäß einem oder mehreren der Ansprüche 1 bis 7 und/oder ihre pharmazeutisch verwendbaren Salze, Tautomere und Stereoisomere, einschließlich deren Mischungen in allen Verhältnissen, und mindestens einen weiteren Arzneimittelwirkstoff.

11. Verwendung von Verbindungen gemäß einem oder mehreren der Ansprüche 1 bis 7 und/oder ihre physiologisch unbedenklichen Salze, Salze und Solvate zur Herstellung eines Arzneimittels zur Behandlung des Typ 1 und Typ 2 Diabetes.

12. Verwendung von Verbindungen gemäß einem oder mehreren der Ansprüche 1 bis 7 und/oder ihre physiologisch unbedenklichen Salze, Tautomere und Stereoisomere zur Herstellung eines Arzneimittels zur Blutzuckersenkung.

13. Verwendung von Verbindungen gemäß einem oder mehreren der Ansprüche 1 bis 7 und/oder ihre physiologisch unbedenklichen Salze, Tautomere und Stereoisomere und einem weiteren Arzneimittelwirkstoff zur Herstellung eines Arzneimittels zur Behandlung des Typ 1 und Typ 2 Diabetes.

14. Set (Kit), bestehend aus getrennten Packungen von
(a) einer wirksamen Menge an einer Verbindung der Formel I gemäß einem oder mehreren der Ansprüche 1 bis 7 und/oder ihrer pharmazeutisch verwendbaren Salze, Tautomere und Stereoisomere, einschließlich deren Mischungen in allen Verhältnissen, und
(b) einer wirksamen Menge eines weiteren Arzneimittelswirkstoffs.

15. Verwendung von Verbindungen gemäß einem oder mehreren der Ansprüche 1 bis 7 und/oder ihre physiologisch unbedenklichen Salze, Tautomere und Stereoisomere und einem weiteren Arzneimittelwirkstoff zur Herstellung eines Arzneimittels zur Blutzuckersenkung.

16. Die Verbindung
7-(1-Methoxy-D-glucopyranos-1-yl)-1-(4-methoxy-phenyl)-3,4-dihydro-naphthalin ("A6")
sowie ihre pharmazeutisch verwendbaren Salze, Tautomere und
Stereoisomere, einschließlich deren Mischungen in allen Verhältnissen.

## Claims

1. Compounds of the formula I 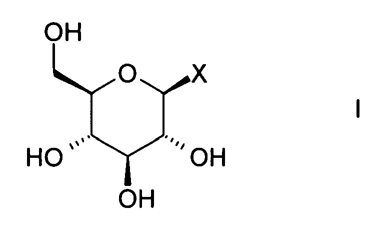 I
in which
X denotes 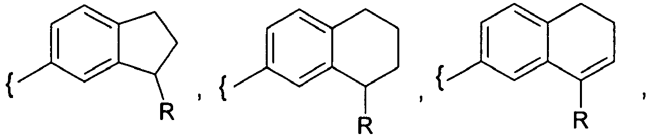 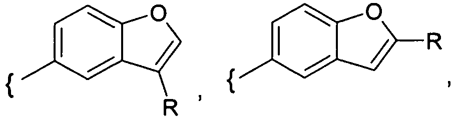 or 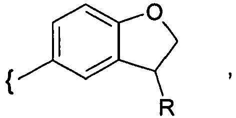 where the 6-membered aromatic ring may be mono- or disubstituted by Hal, OR⁵, NR⁵R⁶, CN, COOR⁵, CONR⁵R⁶, -OCOA, NR⁵COR⁶ and/or NR⁵SO₂A,
R denotes Carb, Ar or Het,
R⁵, R⁶ each, independently of one another, denote H or A,
Carb denotes cycloalkyl having 3-7 C atoms,
Ar denotes phenyl, naphthyl or biphenyl, each of which is unsubstituted or mono-, di-, tri-, tetra- or pentasubstituted by Hal, A, benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)pA and/or -(CR⁵R⁶)ₘ-COOR⁵,
Het denotes a mono- or bicyclic saturated, unsaturated or aromatic heterocycle having 1 to 4 N, O and/or S atoms, which may be mono-, di- or trisubstituted by Hal, A, benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)ₚA, -(CR⁵R⁶)ₘ-COOR⁵, =S, =NR¹ and/or =O (carbonyl oxygen),
A denotes unbranched or branched alkyl having 1-10 C atoms, in which one, two or three CH₂ groups may be replaced, independently of one another, by O, S, SO, SO₂, NR⁵, -C≡C-and/or by -CH=CH- groups and/or in which, in addition, 1-7 H atoms may be replaced by F and/or Cl, or cycloalkyl having 3-7 C atoms,
Hal denotes F, Cl, Br or I,
m denotes 0, 1, 2 or 3,
p denotes 0, 1 or 2,
and pharmaceutically usable salts, tautomers and stereoisomers thereof, including mixtures thereof in all ratios.

2. Compounds according to Claim 1 in which
R⁵, R⁶ each, independently of one another, denote H or methyl, and pharmaceutically usable salts, tautomers and stereoisomers thereof, including mixtures thereof in all ratios.

3. Compounds according to Claim 1 or 2 in which
R denotes Ar,
and pharmaceutically usable salts, tautomers and stereoisomers thereof, including mixtures thereof in all ratios.

4. Compounds according to one or more of Claims 1-3 in which
Ar denotes phenyl which is unsubstituted or mono-, di- or trisubstituted by Hal, A and/or OR⁵,
and pharmaceutically usable salts, tautomers and stereoisomers thereof, including mixtures thereof in all ratios.

5. Compounds according to one or more of Claims 1-4 in which
A denotes unbranched or branched alkyl having 1-6 C atoms, in which 1-7 H atoms may be replaced by F and/or Cl,
and pharmaceutically usable salts, tautomers and stereoisomers thereof, including mixtures thereof in all ratios.

6. Compounds according to Claim 1
in which
X denotes 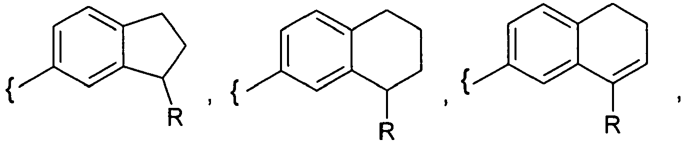 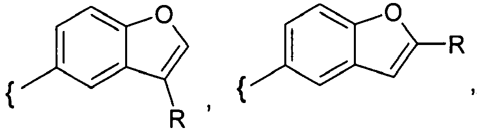 or 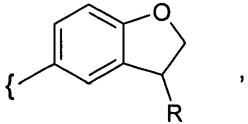
R denotes Ar,
R⁵ denotes H or A,
Ar denotes phenyl which is unsubstituted or mono-, di- or trisubstituted by Hal, A and/or OR⁵,
A denotes unbranched or branched alkyl having 1-6 C atoms, in which 1-7 H atoms may be replaced by F and/or Cl,
Hal denotes F, Cl, Br or I,
and pharmaceutically usable salts, tautomers and stereoisomers thereof, including mixtures thereof in all ratios.

7. Compounds according to Claim 1 selected from the group
| No. | Name and/or structure |
|---|---|
| "A1" | 6-(β-D-Glucopyranos-1-yl)-1-(4-methoxyphenyl)indane |
| "A2" | 6-(β-D-Glucopyranos-1-yl)-1S-(4-methoxyphenyl)indane |
| "A3" | 6-(β-D-Glucopyranos-1R-yl)-1R-(4-methoxyphenyl)indane |
| "A4" | 6-(β-D-Glucopyranos-1-yl)-1-(4-ethoxyphenyl)indane |
| "A5" | 7-(β-D-Glucopyranos-1-yl)-1-(4-methoxyphenyl)-1,2,3,4-tetrahydronaphthalene |
| "A7" | 5-(β-D-Glucopyranos-1-yl)-3-(4-methoxyphenyl)benzofuran |
| "A8" | 5-(β-D-Glucopyranos-1-yl)-2-(4-methoxyphenyl)benzofuran |
| "A9" | 5-(β-D-Glucopyranos-1-yl)-3-(4-methoxyphenyl)-2,3-dihydrobenzofuran |
| "A10" | 5-(β-D-Glucopyranos-1-yl)-3-(4-ethoxyphenyl)benzofuran |
| "A11" | 5-(β-D-Glucopyranos-1-yl)-3-(4-ethoxyphenyl)-2,3-dihydrobenzofuran |
and pharmaceutically usable salts, tautomers and stereoisomers thereof, including mixtures thereof in all ratios.

8. Process for the preparation of compounds of the formula I according to Claims 1-7 and pharmaceutically usable salts, tautomers and stereoisomers thereof, **characterised in that**
R¹ is cleaved off from a compound of the formula II 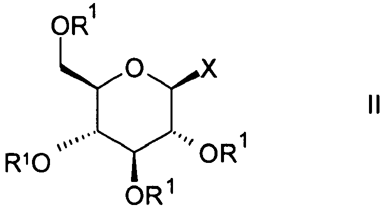 in which
X has the meaning indicated in Claim 1,
and R¹ denotes a hydroxyl-protecting group,
and/or
a base or acid of the formula I is converted into one of its salts.

9. Medicaments comprising at least one compound of the formula I according to one or more of Claims 1 to 7 and/or pharmaceutically usable salts, tautomers and stereoisomers thereof, including mixtures thereof in all ratios, and optionally excipients and/or adjuvants.

10. Medicaments comprising at least one compound of the formula I according to one or more of Claims 1 to 7 and/or pharmaceutically usable salts, tautomers and stereoisomers thereof, including mixtures thereof in all ratios, and at least one further medicament active compound.

11. Use of compounds according to one or more of Claims 1 to 7 and/or physiologically acceptable salts, salts and solvates thereof for the preparation of a medicament for the treatment of type 1 and type 2 diabetes.

12. Use of compounds according to one or more of Claims 1 to 7 and/or physiologically acceptable salts, tautomers and stereoisomers thereof for the preparation of a medicament for lowering blood sugar.

13. Use of compounds according to one or more of Claims 1 to 7 and/or physiologically acceptable salts, tautomers and stereoisomers thereof and a further medicament active compound for the preparation of a medicament for the treatment of type 1 and type 2 diabetes.

14. Set (kit) consisting of separate packs of
(a) an effective amount of a compound of the formula I according to one or more of Claims 1 to 7 and/or pharmaceutically usable salts, tautomers and stereoisomers thereof, including mixtures thereof in all ratios,
and
(b) an effective amount of a further medicament active compound.

15. Use of compounds according to one or more of Claims 1 to 7 and/or physiologically acceptable salts, tautomers and stereoisomers thereof and a further medicament active compound for the preparation of a medicament for lowering blood sugar.

16. The compound
7-(1-methoxy-D-glucopyranos-1-yl)-1-(4-methoxyphenyl)-3,4-dihydronaphthalene ("A6")
and pharmaceutically usable salts, tautomers and stereoisomers thereof, including mixtures thereof in all ratios.

## Revendications

1. Composés de la formule I 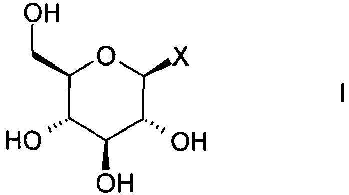 dans laquelle
X représente 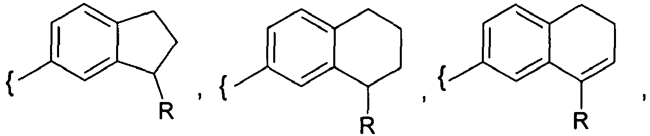 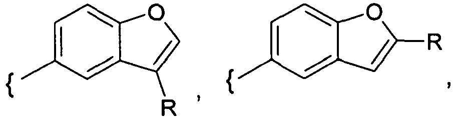 ou 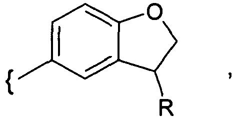 où le cycle aromatique à 6 éléments peut être mono- ou disubstitué par Hal, OR⁵, NR⁵R⁶, CN, COOR⁵, CONR⁵R⁶, -OCOA, NR⁵COR⁶ et/ou NR⁵SO₂A,
R représente Carb, Ar ou Het,
R⁵, R⁶ représentent, chacun indépendamment de l'autre, H ou A,
Carb représente cycloalkyle comportant 3-7 atomes de C,
Ar représente phényle, naphtyle ou biphényle dont chacun est non substitué ou mono-, di-, tri-, tétra- ou pentasubstitué par Hal, A, benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)ₚA et/ou -(CR⁵R⁶)ₘ-COOR⁵,
Het représente un hétérocycle mono- ou bicyclique saturé, non saturé ou aromatique comportant de 1 à 4 atomes de N, de O et/ou de S, lequel peut être mono-, di- ou trisubstitué par Hal, A, benzyl, OR⁵, NR⁵R⁶, NO₂, CN, CONR⁵R⁶, NR⁵COA, OCOA, NR⁵CONR⁵R⁶, NR⁵SO₂A, CHO, COA, SO₂NR⁵R⁶, S(O)pA, -(CR⁵R⁶)ₘ-COOR⁵, =S, =NR¹ et/ou =O (oxygène carbonyle),
A représente alkyle non ramifié pu ramifié comportant 1-10 atomes de C, où un, deux ou trois groupes CH₂ peut/peuvent être remplacé(s), indépendamment les uns des autres, par O, S, SO, SO₂, NR⁵, -C≡C- et/ou par des groupes -CH≡CH-et/ou où, en outre, 1-7 atomes de H peut/peuvent être remplacé(s) par F et/ou Cl, ou cycloalkyle comportant 3-7 atomes de C,
Hal représente F, Cl, Br ou I,
m représente 0, 1, 2 ou 3,
p représente 0, 1 ou 2,
et leurs sels, tautomères et stéréoisomères pharmaceutiquement utilisables, y compris leurs mélanges selon toutes proportions.

2. Composés selon la revendication 1 dans lesquels
R⁵, R⁶ représentent, chacun indépendamment de l'autre, H ou méthyle,
et leurs sels, tautomères et stéréoisomères pharmaceutiquement utilisables, y compris leurs mélanges selon toutes proportions.

3. Composés selon la revendication 1 ou 2 dans lesquels
R représente Ar,
et leurs sels, tautomères et stéréoisomères pharmaceutiquement utilisables, y compris leurs mélanges selon toutes proportions.

4. Composés selon une ou plusieurs des revendications 1-3 dans lesquels
Ar représente phényle qui est non substitué ou mono-, di- ou trisubstitué par Hal, A et/ou OR⁵,
et leurs sels, tautomères et stéréoisomères pharmaceutiquement utilisables, y compris leurs mélanges selon toutes proportions.

5. Composés selon une ou plusieurs des revendications 1-4 dans lesquels
A représente alkyle non ramifié ou ramifié comportant 1-6 atomes de C, où 1-7 atomes de H peut/peuvent être remplacé(s) par F et/ou Cl,
et leurs sels, tautomères et stéréoisomères pharmaceutiquement utilisables, y compris leurs mélanges selon toutes proportions.

6. Composés selon la revendication 1
dans lesquels
X représente 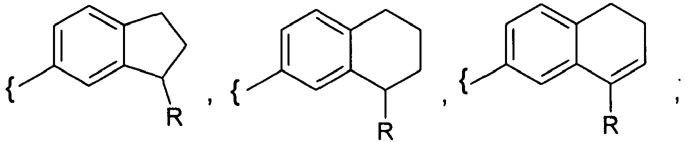 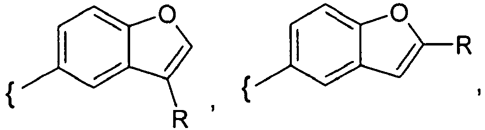 ou 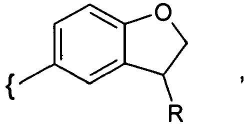
R représente Ar,
R⁵ représente H ou A,
Ar représente phényle qui est non substitué ou mono-, di- ou trisubstitué par Hal, A et/ou OR⁵,
A représente alkyle non ramifié ou ramifié comportant 1-6 atomes de C, où 1-7 atomes de H peut/peuvent être remplacé(s) par F et/ou Cl,
Hal représente F, Cl, Br ou I,
et leurs sels, tautomères et stéréoisomères pharmaceutiquement utilisables, y compris leurs mélanges selon toutes proportions.

7. Composés selon la revendication 1 choisis parmi le groupe
| No. | Nom et/ou structure |
|---|---|
| "A1" | 6-(β-D-Glucopyranos-1-yl)-1-(4-méthoxyphényl)indane |
| "A2" | 6-(β-D-Glucopyranos-1-yl)-1S-(4-méthoxyphényl)indane |
| "A3" | 6-(β-D-Glucopyranos-1-yl)-1R-(4-méthoxyphényl)indane |
| "A4" | 6-(β-D-Glucopyranos-1-yl)-1-(4-éthoxyphényl)indane |
| "A5" | 7-(β-D-Glucopyranos-1-yl)-1-(4-méthoxyphényl)-1,2,3,4-tétrahydronaphtalène |
| "A7" | 5-(β-D-Glucopyranos-1-yl)-3-(4-méthoxy-phényl)benzofurane |
| "A8" | 5-(β-D-Glucopyranos-1-yl)-2-(4-méthoxy-phényl)benzofurane |
| "A9" | 5-(β-D-Glucopyranos-1-yl)-3-(4-méthoxyphényl)-2,3-dihydrobenzofurane |
| "A10" | 5-(β-D-Glucopyranos-1-yl)-3-(4-éthoxy-phényl)benzofurane |
| "A11" | 5-(β-D-Glucopyranos-1-yl)-3-(4-éthoxyphényl)-2,3-dihydrobenzofurane |
et leurs sels, tautomères et stéréoisomères pharmaceutiquement utilisables, y compris leurs mélanges selon toutes proportions..

8. Procédé pour la préparation of composés de la formule I selon les revendications 1-7 et de leurs sels, tautomères et stéréoisomères pharmaceutiquement utilisables, y compris leurs mélanges selon toutes proportions, **caractérisé en ce que**
R¹ est obtenu par clivage à partir d'un composé de la formule II 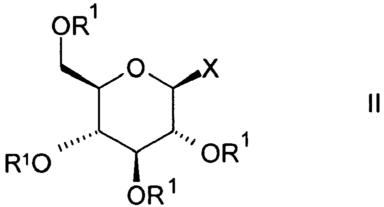 dans laquelle
X présente la signification indiquées selon la revendication 1, et R¹ représente un groupe de protection d'hydroxyle,
et/ou
une base ou un acide de la formule I est converti selon l'un de ses sels.

9. Médicaments comprenant au moins un composé de la formule I selon une ou plusieurs des revendications 1 à 7 et/ou et son/leurs sels, tautomères et stéréoisomères pharmaceutiquement utilisables, y compris leurs mélanges selon toutes proportions et en option, des excipients et/ou adjuvants.

10. Médicaments comprenant au moins un composé de la formule I selon une ou plusieurs des revendications 1 à 7 et/ou et son/leurs sels, tautomères et stéréoisomères pharmaceutiquement utilisables, y compris leurs mélanges selon toutes proportions et au moins un autre composé actif de médicament.

11. Utilisation de composés selon une ou plusieurs des revendications 1 à 7 et/ou de leurs sels, tautomères et stéréoisomères pharmaceutiquement utilisables, y compris leurs mélanges selon toutes proportions pour la préparation d'un médicament pour le traitement de diabètes de type 1 et de type 2.

12. Utilisation de composés selon une ou plusieurs des revendications 1 à 7 et/ou de leurs sels, tautomères et stéréoisomères pharmaceutiquement utilisables, y compris leurs mélanges selon toutes proportions pour diminuer le sucre dans le sang.

13. Utilisation de composés selon une ou plusieurs des revendications 1 à 7 et/ou de leurs sels, tautomères et stéréoisomères pharmaceutiquement utilisables, y compris leurs mélanges selon toutes proportions pour le traitement of de diabètes de type 1 et de type 2.

14. Ensemble (kit) constitué de packs séparés de
(a) une quantité efficace d'un composé de la formule I selon une ou plusieurs des revendications 1 à 7 et/ou de leurs sels, tautomères et stéréoisomères pharmaceutiquement utilisables, y compris leurs mélanges selon toutes proportions,
et
(b) une quantité efficace d'un autre composé actif de médicament.

15. Utilisation de composés selon une ou plusieurs des revendications 1 à 7 et/ou de leurs sels, tautomères et stéréoisomères pharmaceutiquement utilisables, y compris leurs mélanges selon toutes proportions pour la préparation d'un médicament pour diminuer le sucre dans le sang.

16. Le composé
7-(1-méthoxy-D-glucopyranos-1-yl)-1-(4-méthoxyphényl)-3,4-dihydronaphtalène ("A6")
et ses sels, tautomères et stéréoisomères pharmaceutiquement utilisables, y compris leurs mélanges selon toutes proportions.
